(19) Europäisches Patentamt / European Patent Office / Office européen des brevets

(11) **EP 3 705 115 B1**

(12) **EUROPEAN PATENT SPECIFICATION**

(45) Date of publication and mention
of the grant of the patent:
**30.06.2021 Bulletin 2021/26**

(51) Int Cl.:
*A61K 9/14* *(2006.01)*          *A61K 9/16* *(2006.01)*
*A61K 31/4965* *(2006.01)*          *A61P 9/12* *(2006.01)*

(21) Application number: **19174552.0**

(22) Date of filing: **15.05.2019**

(54) **COMPOSITION CONTAINING SELEXIPAG**

ZUSAMMENSETZUNG ENTHALTEND SELEXIPAG

COMPOSITION CONTENANT DU SELEXIPAG

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.03.2019 IN 201911008942**

(43) Date of publication of application:
**09.09.2020 Bulletin 2020/37**

(73) Proprietor: **Alfred E. Tiefenbacher (GmbH & Co.
KG)
22767 Hamburg (DE)**

(72) Inventors:
• **Gujjar, Chaitanya Yogananda
502 319 Telangana (IN)**
• **Staver, Ruslan
22767 Hamburg (DE)**
• **Tejeswararao, Budumuru
502 319 Telangana (IN)**
• **Donga, Nani Prasad
502 319 Telangana (IN)**
• **Bandla, Srimannarayana
502 319 Telangana (IN)**
• **Rallabandi, Bala Ramesha Chary
502 319 Telangana (IN)**
• **Schlehahn, Hendrik
22767 Hamburg (DE)**

(74) Representative: **Hamm&Wittkopp Patentanwälte
PartmbB
Jungfernstieg 38
20354 Hamburg (DE)**

(56) References cited:
**WO-A1-2017/029594          WO-A1-2017/121806
WO-A1-2018/015974**

• **"Amorphous Selexipag Formulations",
RESEARCH DISCLOSURE, KENNETH MASON
PUBLICATIONS, HAMPSHIRE, UK, GB, vol. 618,
no. 27, October 2015 (2015-10), page 2,
XP007144400, ISSN: 0374-4353 [retrieved on
2015-09-09]**

EP 3 705 115 B1

**Description**

The present invention relates to a solid dispersion containing selexipag in a non-crystalline state as well as to a pharmaceutical composition containing the solid dispersion.

Selexipag is a selective prostacyclin ($PGI_2$) receptor (IP receptor) agonist and marketed under the tradename Uptravi® in the form of a film-coated tablet for the treatment of pulmonary arterial hypertension (PAH). Uptravi® contains 200-1600 μg selexipag, whereby the therapy of PAH starts with 200 μg given twice daily, followed by an increase of the daily dose to the highest individually tolerated dose up to a maximum dose of 1600 μ twice daily via increments of 200 μg twice daily, usually at weekly intervals.

Selexipag is insoluble in aqueous solutions at pH 2-4, freely soluble at pH 8 and very soluble at pH 9-12. Hydrolysis of selexipag to the free acid ACT-333679 occurs under both acidic and basic conditions. Three crystalline forms of selexipag have been identified, the Forms I, II and III, which are described in EP-A-2 447 254.

The Uptravi® film-coated tablet is a standard immediate-release tablet that contains D-mannitol, corn starch, low substituted hydroxypropyl cellulose, hydroxypropyl cellulose and magnesium stearate in the tablet core; the film-coating contains hydroxypropyl methylcellulose (hypromellose, HPMC), propylene glycol, titanium dioxide, carnauba wax along with mixtures of iron oxides in order to protect the tablet core from light. The tablet core is prepared by wet-granulation and contains the drug in the crystalline Form I, which is the metastable form (cf. Australian Public Assessment Report for Selexipag, November 2016).

Selexipag is considered to be a BCS (Biopharmaceutics Classification System) Class II drug, i.e. having high permeability and low solubility. Hence, the drug substance needs to be micronized in order to improve solubility, and because of the poor solubility of the drug substance, particle size control is considered to be a critical quality in the manufacture of Uptravi®; the particle size distribution may affect the bioavailability of the drug. In addition, content uniformity of the blends used during the manufacture and of the tablet is considered to be a further critical quality attribute, which is difficult to be achieved because of the very low load of the drug in the tablet.

In order to increase the solubility of selexipag, the use of amorphous drug, or solid dispersions containing amorphous particles of the drug or the drug in molecularly dispersed form (solid solution), i.e. the use of selexipag in a non-crystalline state was considered. WO 2017/029594 describes the preparation of amorphous selexipag by dissolving the drug in a suitable solvent, e.g. acetone, and removing the solvent by, e.g., evaporation, spray-drying or freeze-drying. A solid dispersion may be obtained by dissolving selexipag and a pharmaceutically acceptable carrier, such as polyvinylpyrrolidone (povidone), copovidone, hydroxymethyl cellulose, hydroxypropyl cellulose or hydroxypropyl methylcellulose acetate succinate (HPMC-AS), in a solvent and removing the solvent. In order to increase the hygroscopic stability, the solid dispersion may contain microcrystalline cellulose, colloidal silica, amorphous silica or a mesoporous silica (commercially available under the tradename Syloid®).

WO 2017/121806 discloses that amorphous selexipag has a high tendency to crystallize, which requires that the drug needs to be dispersed in a matrix containing a polymer and/or a silicon-based inorganic adsorbent. Preferably, the polymer is a hydrophilic polymer, e.g. polyvinylpyrrolidone, methacrylic acid copolymers, HPMC-AS or polyvinylcaprolactam/polyvinyl acetate/polyethylene glycol graft copolymer (commercially available under the tradename Soluplus®). Preferred silicon-based inorganic adsorbents include colloidal silica and mesoporous silica (Syloid®). Mesoporous magnesium aluminometasilicate products (commercially available under the tradename Neusilin®) may also be used. The solid dispersion may be prepared by dissolving or dispersing selexipag and the polymer or the silicon-based inorganic adsorbent in a suitable solvent, followed by removing the solvent.

WO 2017/121806 further discloses that, in order to provide granules containing selexipag in a solid dispersion with high content uniformity, the drug should be dissolved or dispersed together with the polymer or silicon-based inorganic adsorbent in a solvent, and the solution/dispersion should be sprayed on the surface of pharmaceutical excipients, followed by granulating the obtained mixture and drying the granules. The granules may be compressed into tablets.

WO 2018/078383 describes a process for the preparation of amorphous selexipag in which selexipag is dispersed in water, and a base, e.g. aqueous ammonia, is added in order to obtain a solution from which amorphous selexipag is precipitated by the addition of an acid, e.g. hydrochloric acid or acetic acid. In addition, the preparation of a solid dispersion containing selexipag in a matrix, preferably in copovidone or Eudragit®, such as Eudragit® E, by dissolving the drug and the matrix former in a suitable solvent, e.g. ethanol, and removing the solvent by evaporation is disclosed.

The article "Amorphous Selexipag Formulations" disclosed in *Research Disclosure* October 2015 (database number 618027) discloses the preparation of a solid dispersion of selexipag in Soluplus® by spray-drying a solution of the drug and the polymer, by granulating a mixture of mannitol and microcrystalline cellulose with a solution of the drug and Soluplus® in ethanol, or by subjecting a mixture of selexipag and Soluplus® to hot-melt extrusion (HME).

WO 2018/015974 discloses the preparation of a solid dispersion of selexipag in copovidone or povidone by HME. In addition, a process for the preparation of a solid dispersion of selexipag in HPMC is disclosed in which the components are dissolved in a mixture of acetone and water and the obtained solution is subjected to spray-drying.

The objective underlying the present invention was the provision of a solid unit dosage form for oral administration that contains selexipag in a non-crystalline state and that shows a high content uniformity. This objective is attained by the subject matter as defined in the claims.

The solid unit dosage form of the present invention is an immediate-release solid unit dosage form for oral administration, preferably an optionally film-coated tablet or a capsule. The solid unit dosage form of the present invention typically contains the drug in a relatively low amount of 2 % by weight or less, based on the total weight of the tablet or, if a film-coating is present, the tablet in core, or based on the total weight of the blend contained in the capsule.

In order to enhance the dissolution rate and, thus, bioavailability of selexipag, the drug is contained in the solid unit dosage form in a non-crystalline state, i.e. either in the form of amorphous particles or in molecularly dispersed form (solid solution). However, non-crystalline selexipag is particularly prone to hydrolysis to the free acid ACT-333679 (Impurity A in the examples). Hence, wet-granulation in the preparation of a solid unit dosage form containing selexipag in a non-crystalline state should be avoided.

High-energy mixing of crystalline selexipag and a pharmaceutical excipient in order to convert crystalline selexipag into its non-crystalline state in a solid dispersion, e.g. ball milling, led, as the wet-granulation process in which crystalline selexipag was dissolved in the granulation liquid, to the formation of the free acid. It was found that crystalline selexipag could be readily converted into its non-crystalline state without hydrolysis by subjecting the drug and a polymer to hot-melt extrusion, which gives an extrudate of non-crystalline selexipag dispersed in the polymer matrix. However, the content uniformity of the extrudate, the milled extrudate and the tablet prepared by compressing the milled extrudate was very poor. In order to improve the content uniformity, the milled extrudate needs to be subjected to a dry-granulation process. Thus, the present invention relates to a process for the preparation of a solid unit dosage form for oral administration containing selexipag in a non-crystalline state, comprising the steps:

> i) subjecting a first mixture containing selexipag, a polymer which is copovidone and optionally a first pharmaceutical excipient to hot-melt extrusion,
> ii) milling the extrudate obtained in step (i) to obtain a milled extrudate,
> iii) mixing the milled extrudate obtained in step (ii) and a second pharmaceutical excipient to obtain a second mixture,
> iv) subjecting the second mixture obtained in step (iii) to compaction,
> v) milling the compacted material obtained in step (iv) to obtain granules,
> vi) optionally mixing the granules obtained in step (v) and a third pharmaceutical excipient to obtain a third mixture, and
> vii) converting the granules obtained in step (v) or the third mixture obtained in step (vi) to the solid unit dosage form.

The polymer used in method step (i) is a non-enteric polymer, copovidone.

Typically, the maximum temperature applied in the hot-melt extrusion is 130°C to 150°C, preferably 135°C to 140°C. The hot-melt extrusion has to be carried out at a temperature that allows the dissolution of crystalline selexipag in the polymer matrix. Typically, the weight ratio of the polymer to selexipag in the first mixture of step (i) is 2 : 1 to 4 : 1, preferably 2.5 : 1 to 3.5 : 1, more preferred about 3 : 1.

The first pharmaceutical excipient, if present, is preferably selected from a sugar alcohol, a monomeric plasticizer and an antioxidant. It was found that the presence of a sugar alcohol enhances the brittleness of the extrudate and, thus, supports the milling process in step (ii). Examples of sugar alcohols include mannitol, xylitol, sorbitol, maltitol, isomalt, maltitol and erythritol. Examples of suitable monomeric plasticizers include triethylcitrate, triacetin, dibutyl sebacate, diethyl phthalate, glyceryl monostearate, glycerin and propylene glycol. Examples of suitable antioxidants include butylated hydroxytoluene (BHT), butylated hydroxyanisole (BHA), sodium metabisulfite, ascorbic acid and propyl gallate.

The second pharmaceutical excipient contained in the second mixture prepared in step (iii) is typically selected from a filler, a disintegrant, a mesoporous inorganic hygroscopic-stability increasing substance and a lubricant.

The third pharmaceutical excipient which may be mixed with the granules obtained in step (v) is typically selected from a disintegrant, a mesoporous inorganic hygroscopic-stability increasing substance and a lubricant.

In order to increase the hygroscopic stability of selexipag, it is preferred that a mesoporous inorganic hygroscopic-stability increasing substance is contained in the second mixture and optionally in the third mixture. A mesoporous material is a material containing pores with diameters between 2 and 50 nm. Suitable mesoporous silica products are commercially available under the tradename Syloid®. As an alternative to mesoporous silica, mesoporous magnesium aluminometasilicate may be used, e.g. the magnesium aluminometasilicates marketed under the tradename Neusilin®. A further alternative is mesoporous magnesium carbonate, which is commercially available under the tradename Upsalite®.

Examples of fillers include microcrystalline cellulose, calcium hydrogen phosphate, lactose (anhydrous or monohydrate), calcium carbonate and mannitol.

Examples of disintegrants include croscarmellose sodium, sodium starch glycolate, polyvinylpolypyrrolidone (crospovidone) and low substituted hydroxypropyl cellulose (L-HPC).

Examples of lubricants include magnesium stearate, calcium stearate, stearic acid, sodium stearyl fumarate and glycerol dibehenate.

According to a preferred embodiment, the first, second and third pharmaceutical excipients are employed in the process of the present invention, wherein:

- the first mixture of step (i) consists of selexipag, copovidone and optionally a first pharmaceutical excipient which is a sugar alcohol,

- the second mixture of step (iv) consists of the milled extrudate and the second pharmaceutical excipient which is a filler, a disintegrant, a mesoporous inorganic hygroscopic-stability increasing substance and a lubricant,

- the third mixture of step (vii) consists of the granules and the third pharmaceutical excipient which is a lubricant and optionally a mesoporous inorganic hygroscopic-stability increasing substance.

Preferably, the disintegrant is croscarmellose sodium, the lubricant is magnesium stearate and the mesoporous inorganic hygroscopic-stability increasing substance is selected from silica and magnesium aluminometasilicate.

Preferably, the filler is selected from mannitol and microcrystalline cellulose.

The present invention further relates to a solid unit dosage form for oral administration containing selexipag in a non-crystalline state prepared by the process according to the present invention. Preferably, the solid unit dosage form is an optionally film-coated tablet or a capsule.

The optionally film-coated tablet may be prepared by subjecting the granules obtained in step (v) or the third mixture obtained in step (vi) to compression and optionally film-coating. The film-coating may be a moisture barrier film-coating in order to increase the hygroscopic stability of the tablet. The capsule may be prepared by filling the granules obtained in step (v) or the third mixture obtained in step (vi) into a capsule.

The optionally film-coated tablet or capsule of the present invention contains selexipag in an amount of 2.0 % by weight of less, preferably 0.10-1.5 % by weight, more preferred 0.14-1.2 % by weight, based on the weight of the granules obtained in step (v) or the third mixture obtained in step (vi) (i.e. based on the weight of the tablet or, if film-coated, the tablet core, or on the weight of the pharmaceutical composition contained in the capsule). The optionally film-coated tablet or capsule of the present invention typically contains the drug in an amount of 200-1600 $\mu$g.

The solid unit dosage form of the present invention is intended for use in a method of treating pulmonary arterial hypertension, either as a combination therapy with an endothelin receptor antagonist and/or a phosphodiesterase type 5 inhibitor or as monotherapy. Since the treatment should be initiated with a daily starting dose of 200 $\mu$g given twice daily, followed by increasing the daily dose in increments of 200 $\mu$g given twice daily to the highest individually tolerated dose up to a maximum dose of 1600 $\mu$g twice daily, the solid unit dosage form preferably contains 200, 400, 600, 800, 1000, 1200, 1400 or 1600 $\mu$g selexipag.

The solid unit dosage form of the present invention may be contained in blister packages, bottles or sachets made for example from PVC, PVDC, PCTFE, COC, PET, PA, Alu, PE or PP and combinations of multilayer films thereof. These packages may comprise a moisture barrier layer and/or they may be packed together with desiccants.

The following examples are intended to further illustrate the present invention.

Examples

Hot-melt extrusion was performed with a Pharma 11 Twin-screw hot-melt extruder from Thermo Fischer Scientific Inc.

In the comparative examples and examples, a mixture of the crystalline Forms I, II and III as described in EP-A-2 447 254 was used as starting material.

XRD measurement details

| | |
|---|---|
| Make/Model: | PANalytical / EMPYREAN or equivalent |
| X-ray tube: | Empyrean Cu LFF HR |
| Wave length: | K-Alpha1, 1.5406 Å |
| Sample mode: | Reflection |
| Spinning: | Yes, 30 RPM |
| Tension: | 45 kV |

(continued)

| | |
|---|---|
| Current: | 40 mA |
| Divergence slit: | Fixed slit ¼° |
| Anti-scatter slit: | Fixed slit ½° |
| Detector: | PIXcel1D-Medipix3 detector(1) |
| Start angle (°2θ): | 2 |
| End angle (°2θ): | 40 |
| Step size (°2θ): | 0.013 |
| Time per step (s): | 290.4450 |
| Scan axis: | Gonio |
| Scan type: | Continuous |
| Scan time: | 1 Hr |

**Sample preparation**

Take the milled extrudes, tablet cores or coated tablets and grind gently to fine powder using agate mortar and pestle. Take sufficient quantity of the powder and fill into the round cavity of the XRD sample holder by 'Back loading technique' using PANalytical sample preparation kit. The sample surface should be smooth and parallel to sample holder surface.

The content uniformity was determined as described according to the following HPLC method:

Mobile phase: Mix 50mM Ammonium dihydrogen phosphate buffer pH 6.0 and Acetonitrile in the ratio of 50:50 v/v and degas.

Chromatographic conditions:

| | |
|---|---|
| Column | : X Bridge C18, 150 x 4.6 mm, 3.5μm or equivalent. |
| Flow rate | : 0.8 mL/minute |
| Detection | : UV, 230 nm |
| Injection Volume | : 10 μL |
| Data acquisition time | : 8 minutes |
| Column temperature | : 40°C |
| Pump mode | : Isocratic |
| Sample cooler | : 10°C |

Diluent: Phosphate buffer pH6.8 and Acetonitrile in the ratio of 50:50 v/v.

Standard Solution: Prepare a standard solution containing 0.06 mg/mL of Selexipag in diluent.

Sample solution: Prepare a sample solution containing 0.06 mg/mL of Selexipag in diluent.

Calculation:

$$\% \text{ Labelled amount of Selexipag} = \frac{At}{As} \times \frac{Ws}{50} \times \frac{3}{50} \times \frac{25}{Lc} \times P$$

At: Area of peak corresponding to Selexipag in test solution chromatogram

As: Average area of peak corresponding to Selexipag obtained from STD-I chromatograms.

Ws: Weight of Selexipag standard used for the preparation of STD-I (mg).

Lc: Label claim of Selexipag (mg)

Calculation of Acceptance value (AV) for content uniformity:

Test the first 10 units for content uniformity and calculate the acceptance value. The requirement for dosage uniformity are met if the Acceptance value of the first 10 tablets is less than or equal to L1 criteria (L1=15).

If the acceptance value is greater than L1 (acceptance value is 15), Test the next 20 units for content uniformity and calculate the acceptance value.

The requirements are met if the final acceptance value of the 30 tablets is less than or equal to L1 and no individual content of the dosage unit is less than (1 - L2 $\times$ 0.01) M and no individual content is more than (1 + L2 $\times$ 0.01) M in calculation of acceptance value under content uniformity. L2 is 25.0.

Condition 1: If mean value of individual contents is between 98.5 and 101.5.
Acceptance value = KS
Condition 2: If mean value of individual contents is less than 98.5.

$$Acceptance\ value = 98.5 - M + KS$$

Condition 3: If mean value of individual contents is greater than 101.5.

$$Acceptance\ value = M - 101.5 + KS$$

where,

M = Mean of individual contents
K = 2.4 for 10 units and K = 2.0 for 30 units, S = Sample standard deviation

The related substances are measured as described according to the following HPLC method.

Mobile phase-A: Mix 50mM Ammonium dihydrogen phosphate buffer pH 6.0 and Acetonitrile in the ratio of 95:5 v/v and degas.

Mobile phase-B: Mix Acetonitrile and Methanol in the ratio of 85:15 v/v and degas.

Diluent: Phosphate buffer pH6.8 and Acetonitrile in the ratio of 50:50 v/v.

Chromatographic conditions:

| | |
|---|---|
| Column | : Zorbax SB-Phenyl C18, 250 x 4.6 mm, 5.0μm or equivalent. |
| Flow rate | : 1.0 mL/minute |
| Detection | : UV, 230 nm |
| Injection Volume | : 10 μL |
| Data acquisition time | : 55 minutes |
| Column temperature | : 40°C |
| Pump mode | : Gradient |
| Sample cooler | : 10°C |

Gradient program :

| Time (in min) | Mobile phase-A (%) | Mobile phase-B (%) |
|---|---|---|
| 0.01 | 65 | 35 |
| 8.0 | 60 | 40 |
| 25 | 35 | 65 |
| 40 | 35 | 65 |
| 42 | 65 | 35 |
| 50 | 65 | 35 |

Standard Solution: Prepare a standard solution containing 0.0008 mg/mL of Selexipag in diluent.

Detector sensitivity Solution: 0.08ppm solution of standard.

Sample solution: Prepare a sample solution containing 0.16 mg/mL of Selexipag in diluent.

$$\% \text{ of impurities } = \frac{At}{As} \times \frac{Ws}{100} \times \frac{4}{100} \times \frac{4}{100} \times \frac{200}{Wt} \times \frac{Avg. wt}{Lc} \times P \times CF$$

At: Area of peak corresponding to Selexipag in test solution chromatogram
As: Average area of peak corresponding to Selexipag obtained from STD-I chromatograms
Ws: Weight of Selexipag standard used for the preparation of STD-I (mg).
Lc: Label claim of Selexipag (mg)
Wt: Weight of sample taken
P: Potency of standard
CF: Correction factor for known impurities

The related substances abbreviations are corresponding to the following chemical names:

Imp-A: {4-[(5,6-Diphenyl-pyrazin-2-yl)-isopropyl-amino]-butoxy}-acetic acid (SXP-II)
Imp-B: {4-[(5,6-Diphenyl-pyrazin-2-yl)-isopropyl-amino]-butoxy}-acetic acid tert-butyl ester
Imp-C: N-(2-{4-[(5,6-Diphenyl-pyrazin-2-yl)-amino]-butoxy}-acetyl)-methane sulfonamide (Desmethyl selexipag)
Imp-D: 2-{4-[(5,6-Diphenyl-pyrazin-2-yl)-isopropyl-amino]-butoxy}-acetamide (Amide)

Comparative Examples 1 and 2

| | Com. Ex. 1 [mg] | Com. Ex. 2 [mg] |
|---|---|---|
| **Stage-A (Drug solution)** | | |
| Selexipag | 1.619 | 1.619 |
| Sodium Carbonate, Anhydrous | 0.500 | 0.500 |
| Povidone (Plasdone™ K 29/32) | 3.000 | - |
| Hypromellose (Methocel™ E5LV) | - | 3.000 |
| Water, Purified | 35.000 | 35.000 |
| **Stage-B (Granulation)** | | |
| Magnesium Aluminometasilicate (Neusilin US2) | 69.981 | 69.981 |
| **Stage-C (Blending)** | | |
| Mannitol (Pearlitol® 200SD) | 54.890 | 54.890 |
| Sodium Starch Glycolate (Explotab®) | 4.000 | 4.000 |
| **Stage-D (Lubrication for Compression)** | | |
| Magnesium stearate (Ligamed MF-2-V) | 1.010 | 1.010 |
| **Core tablet weight** | 135.000 | 135.000 |

**Procedure:**

1. Weighed quantity of Sodium carbonate was dissolved in purified water.

2. Weighed quantity of Selexipag was dissolved in Sodium carbonate solution.

3. Povidone or Hypromellose was dissolved in the solution of step 2 and the drug solution was added to Neusilin.

4. The granules were dried at 60°C using Rapid dryer to get the LOD not more than 5%m/m at 105°C.

5. The dried granules were sifted through mesh #30.

6. Mannitol and Sodium Starch Glycolate were sifted through mesh #30.

7. Magnesium stearate was sifted through mesh #40.

8. Granules along with the materials of steps 6 and 7 were blended and lubricated for 5 minutes.

9. The lubricated blend was compressed with 8 mm, round, standard concave punches.

**Table 1. Related substances results of core tablets**

| | Comp. Ex. 1 | | Comp. Ex. 2 | | |
|---|---|---|---|---|---|
| | Initial | 40°C/75%RH 1M (Alu-Alu) | Initial | 50°C/75%RH, 5 Days Open | 60°C, 2 Days Glass vial |
| Related Substances (%m/m by HPLC) | | | | | |
| Imp-A | 3.44 | 19.13 | 0.25 | 13.03 | 6.55 |
| Imp-B | ND | ND | ND | ND | ND |
| Imp-C | 0.15 | 0.07 | 0.11 | 0.09 | 0.03 |
| Imp-D | ND | ND | ND | ND | ND |
| Unspecified impurity | 0.06 | 0.05 | 0.13 | 0.07 | 0.06 |
| Total impurities | 3.68 | 21.11 | 0.51 | 15.68 | 7.04 |

Crystalline selexipag was completely converted into the non-crystalline state. On stability, after 1 month at 40°C/75%RH, the impurity levels are further increased in Alu-Alu blister packed tablets. The formulation with HPMC (Comparative Example 2) has low degradation levels at the initial stage but under stress conditions, the impurity levels increased.

Comparative Example 3

| Stage-A (Blending) | [mg] |
|---|---|
| Selexipag-Syloid (1 : 5) Ball milled mixture | 9.720 |
| Cellulose, Microcrystalline (Comprecel® M 102) | 65.380 |
| Mannitol (Pearlitol®200SD) | 56.000 |
| **Stage-B (Lubrication and Slugging)** | |
| Magnesium stearate (Ligamed MF-2-V) | 0.700 |
| **Stage-C (Blending and Compression)** | |
| Croscarmellose sodium (Ac-Di-Sol®) | 1.715 |
| Magnesium stearate (Ligamed MF-2-V) | 0.476 |
| **Tablet weight** | **135.000** |

**Procedure:**

1. Ball milled Selexipag-Syloid (1 : 5) mixture, Cellulose, Microcrystalline and Mannitol were sifted through mesh #30.

2. Materials of step 1 were blended for 10 minutes.

3. Magnesium stearate was sifted through mesh #40.

4. Sifted Magnesium stearate of step 3 was added to the blend of step 2 and lubricated for 5 minutes.

5. Lubricated blend was compressed into slugs with 16 mm, round, flat punches.

6. Slugs were milled through 1575 μ screen using Quadro Comil® and passed through mesh #30.

7. Extra-granular Croscarmellose sodium was sifted through mesh #30 and Magnesium stearate was sifted through mesh #40.

8. Granules along with the materials of step 7 were blended for 5 minutes.

9. Lubricated blend was compressed with 7 mm, round, standard concave punches.

**Table 2. Related substances results of tablet**

|  | Related substances (% m/m by HPLC) |
|---|---|
| Imp-A | 11.36 |
| Imp-B | ND |
| Imp-C | ND |
| Imp-D | 1.23 |
| Unspecified impurity | 1.92 |
| Total impurities | 20.52 |

The diffractogram of Ball milled Selexipag-Syloid mixture (1 : 5) did not exhibit crystalline Selexipag reflexes by XRD qualitative analysis. Impurity levels are at high levels after the preparation of the tablet.

Comparative Example 4

| Stage-A (Hot melt extrusion) | [mg] |
|---|---|
| Selexipag | 1.619 |
| Copovidone (Kollidon® VA 64) | 4.857 |
| Triethyl citrate (Citrofol® A1) | 0.070 |
| **Stage-B (Blending)** | |
| Mannitol (Pearlitol® 200SD) | 71.950 |
| Cellulose, Microcrystalline (Comprecel® M 102) | 53.000 |
| Croscarmellose sodium (Ac-Di-Sol®) | 2.430 |
| **Stage-C (Lubrication)** | |
| Magnesium stearate (Ligamed MF-2-V) | 1.000 |

(continued)

| Stage-C (Lubrication) | |
|---|---|
| **Core tablet weight** | **135.000** |
| **Stage-D (Coating)** | |
| Opadry Brown 03F565239 | 5.000 |
| Water, Purified | 36.666 |
| **Coated tablet weight** | **140.000** |

**Procedure:**

1. Accurately dispensed quantity of Selexipag and Copovidone were sifted through mesh #30.
2. The materials of step 1 were loaded into double cone blender and blended for 10 minutes.
3. Triethyl citrate was added to the materials of step 2 and mixed.
4. The mixture of step 3 was hot melt extruded by using following screw design and parameters

| EXT | 7.5 FS | 0 | 0 | 0 | 0 | 0 | 90 | 0 | 90 | 0 | 90 | 90 | 90 | 90 | 90 | 90 | 0 | 90 | 0 | 2FS | 0 | 0 | 0 | 90 | 0 | 0 | 0 | 0 | 4FS | 0 | 0 | 0 | 0 | 90 | 0 | 90 | 0 | 90 | 0 | 16FS |
|---|---|---|---|---|---|---|---|---|---|---|---|---|---|---|---|---|---|---|---|---|---|---|---|---|---|---|---|---|---|---|---|---|---|---|---|---|---|---|---|---|
| 1.5D | 1D | F 60 | F 60 | F 60 | F 60 | F 30 | F 30 | A 90 | A 90 | A 90 | F 60 | F 60 | F 60 | F 60 | F 60 | F 30 | F 30 | F 30 | 1D | F 60 | F 60 | A 90 | A 90 | F 60 | F 60 | F 60 | 1D | F 60 | F 60 | F 60 | F 30 | F 30 | F 30 | F 30 | F 30 | F 30 | 1D |

| | Zone temperatures (°C) | | | | | | | | Die Melt (°C) | Die bar | Torque (%) | Screw rpm |
|---|---|---|---|---|---|---|---|---|---|---|---|---|
| | 2 | 3 | 4 | 5 | 6 | 7 | 8 | Die | | | | |
| Set | 40 | 80 | 120 | 135 | 135 | 135 | 135 | 138 | | | | |
| Time (min) | Actual | Actual | Actual | Actual | Actual | Actual | Actual | Actual | | | | |
| Initial | 40 | 80 | 120 | 135 | 135 | 135 | 135 | 138 | 137 | 0 | 39 | 200 |
| 5 | 40 | 80 | 120 | 135 | 135 | 135 | 135 | 138 | 139 | 4 | 33 | 200 |
| 8 | 40 | 80 | 120 | 135 | 135 | 135 | 135 | 138 | 140 | 1 | 21 | 200 |
| 10 | 40 | 80 | 120 | 135 | 135 | 135 | 135 | 138 | 141 | 0 | 13 | 200 |

5. Extrudes were milled at 4000 rpm through 1016 μ screen using Quadro Comil®.
6. Extra-granular Mannitol and Cellulose, Microcrystalline were sifted through mesh #30.
7. Magnesium stearate was sifted through mesh #40.
8. Milled extrudes of step 5 along with the materials of step 6 were blended for 10 minutes using Double cone blender.
9. Sifted Magnesium stearate of step 7 was added to the materials of step 8 and lubricated for 5 minutes.
10. The lubricated blend was compressed with 7 mm, round, standard concave punches.
11. Core tablets were coated using Opadry Brown 03F565239.

**Table 3. Related substances results of coated tablet**

|  | Initial | 40°C/75%RH, Alu-Alu blister | | |
|---|---|---|---|---|
|  |  | 1M | 2M | 3M |
| Imp-A | 0.08 | 0.14 | 0.19 | 0.22 |
| Imp-B | ND | ND | ND | ND |
| Imp-C | 0.01 | ND | ND | ND |
| Imp-D | ND | 0.02 | ND | ND |
| Unspecified impurity | 0.03 | 0.02 | 0.07 | 0.17 |
| Total impurities | 0.48 | 0.22 | 0.26 | 0.56 |

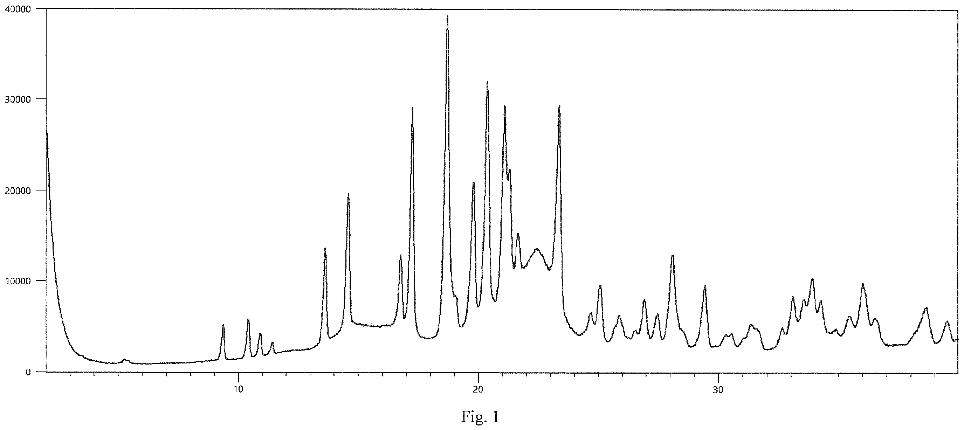

Crystalline form of Selexipag is completely converted into non-crystalline form, as evidenced by the absence of the characteristic XRD peaks for forms I, II and III in the XRD pattern obtained for the tablet cores (Figure 1). The impurity levels are in desired limits at initial stage and up to 3 months of stability at 40°C/75% RH in Alu-Alu blister pack.

Comparative Example 5

| Stage-A (Hot melt extrusion) | [mg] |
|---|---|
| Selexipag | 1.619 |
| Hypromellose (Affinisol™ HME 15LV) | 4.861 |
| Xylitol (Xylisorb® P90) | 0.490 |
| Stage-B (Blending) | |
| Mannitol (Pearlitol® 200SD) | 71.950 |
| Cellulose, Microcrystalline (Comprecel® M 102) | 53.000 |
| Croscarmellose sodium (Ac-Di-Sol®) | 2.430 |
| Stage-C (Lubrication) | |
| Magnesium stearate (Ligamed MF-2-V) | 1.000 |
| Core tablet weight | 135.000 |
| Stage-D (Coating) | |
| Opadry Brown 03F565239 | 5.000 |
| Water, Purified | 36.666 |
| Coated tablet weight | 140.000 |

**Procedure:**

1. Accurately dispensed quantity of Selexipag, Hydroxypropyl methylcellulose and Xylitol were sifted through mesh #30.
2. The materials of step 1 were manually blended for 10 minutes.
3. The mixture of step 2 was hot melt extruded by using following screw design and parameters,

| EXT | 7.5 FS | 0 | 0 | 0 | 0 | 90 | 0 | 90 | 0 | 90 | 90 | 90 | 90 | 90 | 90 | 0 | 90 | 0 | 2FS | 0 | 0 | 0 | 90 | 0 | 0 | 0 | 0 | 4FS | 0 | 0 | 0 | 0 | 90 | 0 | 90 | 0 | 90 | 0 | 16FS |
|---|---|---|---|---|---|---|---|---|---|---|---|---|---|---|---|---|---|---|---|---|---|---|---|---|---|---|---|---|---|---|---|---|---|---|---|---|---|---|---|
| 1.5D | 1D | F60 | F60 | F60 | F60 | F30 | F30 | A90 | A90 | A90 | F60 | F60 | F60 | F60 | F60 | F30 | F30 | F30 | 1D | F60 | F60 | A90 | A90 | F60 | F60 | F60 | 1D | F60 | F60 | F60 | F30 | F30 | F30 | F30 | F30 | F30 | F30 | 1D |

| | Zone temperatures (°C) | | | | | | | | Die Melt (°C) | Die bar | Torque (%) | Screw rpm |
|---|---|---|---|---|---|---|---|---|---|---|---|---|
| | 2 | 3 | 4 | 5 | 6 | 7 | 8 | Die | | | | |
| Set | 40 | 80 | 120 | 135 | 135 | 135 | 135 | 138 | | | | |
| Time (min) | Actual | Actual | Actual | Actual | Actual | Actual | Actual | Actual | | | | |
| Initial | 40 | 80 | 120 | 135 | 135 | 135 | 135 | 138 | 137 | 18 | 9 | 200 |
| 5 | 40 | 80 | 120 | 135 | 135 | 135 | 135 | 138 | 139 | 32 | 11 | 200 |
| 10 | 40 | 80 | 120 | 135 | 135 | 135 | 135 | 138 | 141 | 37 | 12 | 200 |

4. Extrudes were milled through 1016 μ grated screen at 4000 rpm using Quadro Comil®.

5. The milled extrudes were further milled through 457 μ screen at 4000 rpm using Quadro co-mill.

6. Extra granular Mannitol and Cellulose, Microcrystalline were sifted through mesh a#30.

7. Magnesium stearate was sifted through mesh #40.

8. Milled extrudes of step 5 along with the materials of step 6 were blended for 10 minutes using Double cone blender.

9. Sifted Magnesium stearate of step 7 was added to the materials of step 9 and lubricated for 5 minutes.

10. The lubricated blend was compressed with 7 mm, round, standard concave punches.

11. Core tablets were coated using Opadry Brown 03F565239.

**Table 4. Content uniformity of coated tablets**

| Tablet | Content Uniformity |
|---|---|
| 1 | 82.4 |
| 2 | 103.8 |
| 3 | 132.9 |
| 4 | 83.8 |
| 5 | 89.5 |
| 6 | 97.4 |
| 7 | 94.5 |
| 8 | 98.7 |
| 9 | 93.3 |
| 10 | 82.3 |
| Avg. | **95.9** |
| Min | **82.3** |
| Max | **132.9** |
| SD | **14.93** |
| %RSD | **15.58** |
| AV | **38.43** |

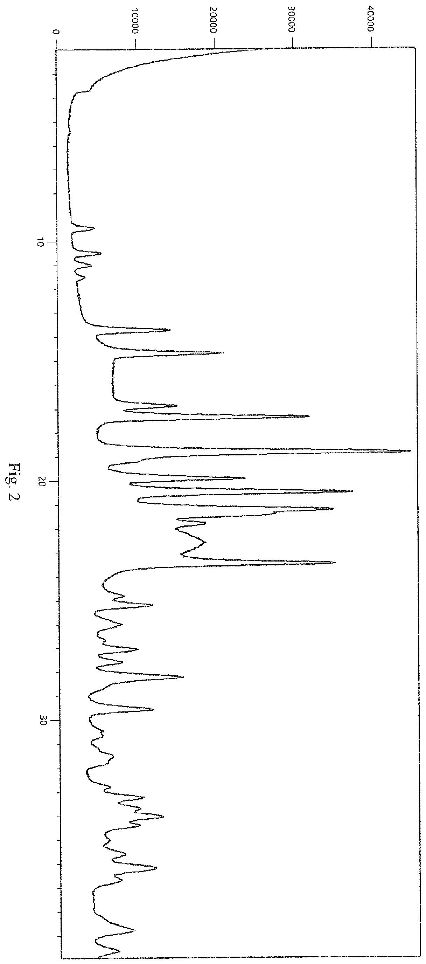

Crystalline selexipag was completely converted into the non-crystalline form, as evidenced by the absence of the characteristic XRD peaks for forms I, II and III in the XRD pattern obtained for the coated tablets (Figure 2). Content uniformity was poor.

Example 1

| Stage-A (Hot melt extrusion) | [mg] |
|---|---|
| Selexipag | 1.619 |
| Copovidone (Kollidon® VA64) | 4.861 |
| **Stage-B (Blending)** | |
| Mannitol (Pearlitol® 200SD) | 71.620 |
| Cellulose, Microcrystalline (Comprecel® M 102) | 53.000 |
| Croscarmellose sodium (Ac-Di-Sol®) | 2.500 |
| **Stage-C (Lubrication for slugging)** | |
| Magnesium stearate (Ligamed MF-2-V) | 0.700 |
| **Stage-C (Lubrication for compression)** | |
| Magnesium stearate (Ligamed MF-2-V) | 0.700 |
| **Core tablet weight** | **135.000** |
| **Stage-D (Coating)** | |
| Opadry Brown 03F565239 | 5.000 |
| Water, Purified | 36.666 |
| **Coated tablet weight** | **140.000** |

**Procedure:**

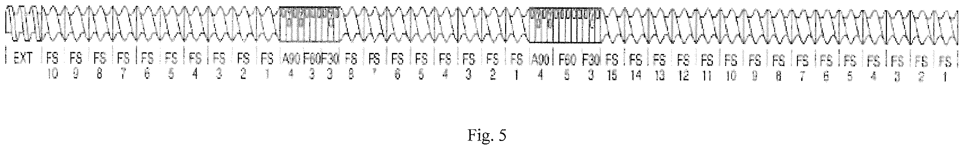

1. Accurately dispensed quantity of Selexipag, Hydroxypropyl methylcellulose and Xylitol were sifted through mesh #25.
2. The materials of step 1 were manually blended for 10 minutes.
3. The mixture of step 3 was hot melt extruded by using following screw design and parameters (Fig. 5)

| | Zone temperatures (°C) | | | | | | | | Die Melt (°C) | Die bar | Torque (%) | Screw rpm |
|---|---|---|---|---|---|---|---|---|---|---|---|---|
| | 2 | 3 | 4 | 5 | 6 | 7 | 8 | Die | | | | |
| Set | 40 | 80 | 120 | 135 | 135 | 135 | 135 | 138 | | | | |
| Time (min) | Actual | Actual | Actual | Actual | Actual | Actual | Actual | Actual | | | | |
| Initial | 40 | 80 | 120 | 135 | 135 | 135 | 135 | 138 | 134 | 6 | 20 | 200 |
| 5 | 40 | 80 | 120 | 135 | 135 | 135 | 135 | 138 | 135 | 5 | 24 | 200 |

4. Extrudes were milled using Quadro Comil® through 813 μ and 457 μ screens at 4000 rpm and passed through mesh #40.
5. Extragranular Mannitol, Cellulose, Microcrystalline and Croscarmellose sodium were sifted through mesh #30.
6. Magnesium stearate was sifted through mesh #40.

7. Milled extrudes of step 4 along with the materials of step 5 were blended for 10 minutes using Double cone blender.

8. Sifted Magnesium stearate of step 6 was added to the materials of step 8 and lubricated for 5 minutes.

9. Lubricated blend of step 8 was slugged using 16 mm, round, flat punches at 40-60 N hardness.

10. Slugs were milled using Multi mill through 2 mm screen with knives forward direction at slow speed.

11. Milled granules of step 10 and sifted Magnesium stearate was transferred into 2 liter Double cone blender and lubricated for 5 minutes at 20 rpm.

12. The lubricated blend was compressed with 7 mm, round, standard concave punches.

13. Core tablets were coated using Opadry Brown 03F565239.

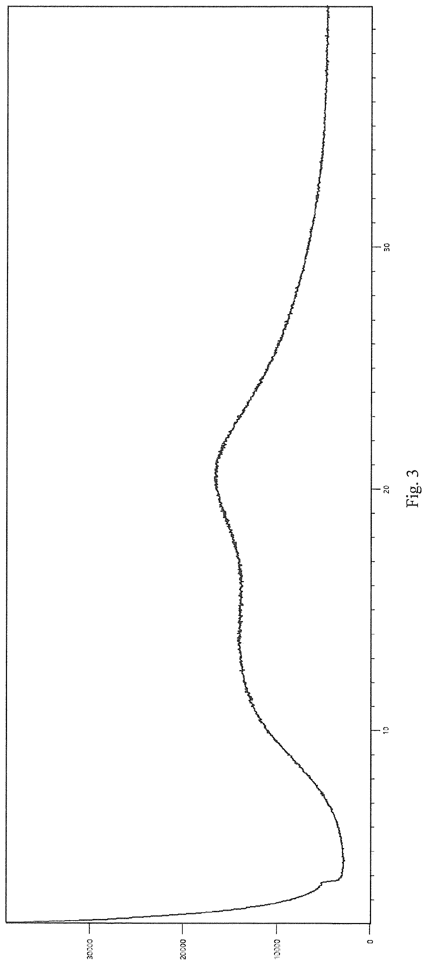

The diffractogram of milled extrudes does not exhibit crystalline Selexipag reflexes in the XRD study (Figure 3). Hence, the API is in a non-crystalline state.

**Table 5. Content uniformity of coated tablets**

| Tablet | Content Uniformity |
|---|---|
| 1 | 104.6 |
| 2 | 102.7 |
| 3 | 105.2 |
| 4 | 98.8 |
| 5 | 94.1 |
| 6 | 104.5 |
| 7 | 93.3 |
| 8 | 106.9 |
| 9 | 101.4 |
| 10 | 105.4 |
| Avg. | **101.7** |
| Min | **93.3** |
| Max | **106.9** |
| SD | **4.8** |
| %RSD | **4.72** |
| AV | **11.72** |

**Table 6. Impurities profile of coated tablets at various conditions***

| Test | Initial (Polybag) | 80°C/ 75% RH 2 Days | 80°C/ 40% RH 2 Days | 70°C/ 75% RH 2 Days | 50°C/75% RH 14 Days | 60°C/75% RH 14 Days | 60°C/40% RH 14 Days | 70°C/5% RH 14 Days |
|---|---|---|---|---|---|---|---|---|
| Impurity-A | 0.08 | 25.42 | 1.53 | 5.77 | 6.67 | 19.23 | 0.61 | 0.09 |
| Impurity-B | ND | ND | ND | ND | ND | ND | ND | ND |
| Impurity-C | ND | ND | ND | ND | ND | ND | ND | ND |
| Impurity-D | 0.01 | 0.51 | 0.09 | 0.13 | 0.08 | 0.22 | 0.03 | 0.02 |
| Maximum Unknown | 0.09 | 3.55 | 0.21 | 0.68 | 0.49 | 1.42 | 0.07 | 0.03 |
| Total Impurities | 0.22 | 30.49 | 2.10 | 6.92 | 7.49 | 23.53 | 0.88 | 0.17 |
| * Tablets are kept in open condition in all the stability environments | | | | | | | | |

Impurity-A levels are increasing in humid conditions irrespective of temperature. However, in presence of high temperatures the impurity-A levels are further increasing (comparing 70°C/75%RH and 80°C/75%RH). Based on the results, it is opined that the moisture is the main reason for instability of the formulation.

Example 2

| Stage-A (Hot melt extrusion) | [mg] |
|---|---|
| Selexipag | 1.619 |
| Copovidone (Kollidon® VA64) | 4.860 |
| **Stage-B (Blending)** | |
| Mannitol (Pearlitol® 200SD) | 102.82 |
| Cellulose, Microcrystalline (Comprecel® M 102) | 20.000 |
| Croscarmellose sodium (Ac-Di-Sol®) | 2.500 |
| Colloidal hydrated Silica (Syloid AL1-FP®) | 1.500 |
| **Stage-C (Lubrication for slugging)** | |
| Magnesium stearate (Ligamed MF-2-V) | 0.700 |
| **Stage-C (Lubrication for compression)** | |
| Magnesium stearate (Ligamed MF-2-V) | 0.700 |
| **Core tablet weight** | **135.00** |
| **Stage-D (Coating)** | |
| Opadry 06A540006 Pink | 5.000 |
| Water, Purified | 36.666 |
| **Coated tablet weight** | **140.00** |

**Procedure:**

1. Accurately dispensed quantity of Selexipag, Maltitol were sifted through mesh #25.
2. The materials of step 1 were manually blended for 10 minutes.
3. The mixture of step 3 was hot melt extruded by using following screw design and parameters (Fig. 5)

| | | Zone temperatures (°C) | | | | | | | | Die Melt (°C) | Die bar | Torque (%) | Screw rpm |
|---|---|---|---|---|---|---|---|---|---|---|---|---|---|
| | | 2 | 3 | 4 | 5 | 6 | 7 | 8 | Die | | | | |
| Set | | 40 | 80 | 120 | 135 | 135 | 135 | 135 | 138 | | | | |
| Time (min) | | Actual | Actual | Actual | Actual | Actual | Actual | Actual | Actual | | | | |
| Initial | | 40 | 80 | 120 | 135 | 135 | 135 | 135 | 138 | 137 | 4 | 20 | 200 |
| 5 | | 40 | 80 | 120 | 135 | 135 | 135 | 135 | 138 | 138 | 5 | 27 | 200 |
| 10 | | 40 | 80 | 120 | 135 | 135 | 135 | 135 | 138 | 137 | 2 | 18 | 200 |

4. Extrudes were milled using Quadro Comil® through 813 μ and 457 μ screen at 4000 rpm.
5. Milled extrudes were passed through mesh #40.
6. Milled extrudes of step 4 along with the materials of step 5 were blended for 10 minutes using Double cone blender.
7. Sifted Magnesium stearate of step 6 was added to the materials of step 8 and lubricated for 5 minutes.

8. Lubricated blend of step 8 was slugged using 16 mm, round, flat punches at 40-60 N hardness.

9. Slugs were milled using Multi mill through 2 mm screen with knives forward direction at slow speed.

10. Milled granules of step 10 and sifted Magnesium stearate was transferred into 2 liter Double cone blender and lubricated for 5 minutes at 20 rpm.

11. The lubricated blend was compressed with 7 mm, round, standard concave punches.

12. Core tablets were coated using Opadry 06A540006 Pink.

**Table 7. Content uniformity of coated tablets**

| Tablet | Content Uniformity |
|--------|--------------------|
| 1 | 103.8 |
| 2 | 90.6 |
| 3 | 96.2 |
| 4 | 101.7 |
| 5 | 92.9 |
| 6 | 89.7 |
| 7 | 98.6 |
| 8 | 101.4 |
| 9 | 100.9 |
| 10 | 94.3 |
| **Avg.** | **97** |
| **Min** | **89.7** |
| **Max** | **103.8** |
| **SD** | **4.98** |
| **%RSD** | **5.14** |
| **AV** | **13.45** |

**Table 8. Related substances results of coated tablet**

| | Initial | 60°C, 2 days in glass vial |
|---|---------|----------------------------|
| Imp-A | 0.11 | 0.65 |
| Imp-B | ND | ND |
| Imp-C | ND | 0.08 |
| Imp-D | 0.01 | 0.02 |
| Unspecified impurity | 0.05 | 0.11 |
| Total impurities | 0.24 | 0.91 |

The diffractogram of milled extrudes does not exhibit crystalline Selexipag reflexes in the XRD study. Hence, the API is in a non-crystalline state.

Examples 3 and 4

| Stage-A (Hot melt extrusion) | [mg] | |
|---|---|---|
| Selexipag | 1.619 | |
| Copovidone (Kollidon® VA64) | 4.860 | |
| Mannitol (Pearlitol® 200SD) | 1.619 | |
| **Stage-B (Blending)** | | |
| Mannitol (Pearlitol® 200SD) | 100.70 | |
| Cellulose, Microcrystalline (Comprecel® M 102) | 20.000 | |
| Croscarmellose sodium (Ac-Di-Sol®) | 2.500 | |
| Colloidal hydrated Silica (Syloid® AL1-FP) | 1.500 | |
| **Stage-C (Lubrication for slugging)** | | |
| Magnesium stearate (Ligamed MF-2-V) | 0.700 | |
| **Stage-C (Lubrication for compression)** | | |
| Colloidal hydrated Silica (Syloid® AL1-FP) | 0.500 | |
| Magnesium stearate (Ligamed MF-2-V) | 1.000 | |
| **Core tablet weight** | **135.000** | |
| **Stage-D (Coating)** | | |
| | **Ex. 3** | **Ex. 4** |
| Opadry Brown 03F565239 | 5.000 | - |
| Opadry AMB II 88A180040 White | - | 5.000 |
| Water, Purified | 36.666 | 20.000 |
| **Coated tablet weight** | **140.000** | **140.000** |

**Procedure:**

1. Accurately dispensed quantity of Selexipag, Copovidone and Mannitol were sifted through mesh #25.
2. The materials of 1 were blended for 10 minutes.
3. The mixture of step 3 was hot melt extruded by using following screw design and parameters (Fig. 5)

| | Zone temperatures (°C) | | | | | | | | Die Melt (°C) | Die bar | Torque (%) | Screw rpm |
|---|---|---|---|---|---|---|---|---|---|---|---|---|
| | 2 | 3 | 4 | 5 | 6 | 7 | 8 | Die | | | | |
| Set | 40 | 80 | 120 | 135 | 135 | 135 | 135 | 138 | | | | |
| Time (min) | Actual | Actual | Actual | Actual | Actual | Actual | Actual | Actual | | | | |
| Initial | 40 | 80 | 120 | 135 | 135 | 135 | 135 | 138 | 134 | 6 | 20 | 200 |
| 5 | 40 | 80 | 120 | 135 | 135 | 135 | 135 | 138 | 135 | 5 | 24 | 200 |
| 10 | 40 | 80 | 120 | 135 | 135 | 135 | 135 | 138 | 135 | 3 | 19 | 200 |

4. Extrudes were milled using Quadro Comil® through 813 and 457 μ screens at 4000 rpm.
5. Extragranular Mannitol, Cellulose, Microcrystalline and Croscarmellose sodium were sifted through mesh #30.
6. Syloid AL1 -FP was sifted through mesh #40.
7. Magnesium stearate was sifted through mesh #40.
8. Milled extrudes of step 4 along with the materials of steps 5 and 6 were blended for 10 minutes using Double cone blender.

9. Sifted Magnesium stearate of step 7 was added to the materials of step 8 and lubricated for 5 minutes.

10. Lubricated blend of step 8 was slugged using 16 mm, round, flat punches at 50-70 N hardness.

11. Slugs were milled using Multi mill through 2 mm screen with knives forward direction at slow speed.

12. Milled granules of step 10 and sifted Magnesium stearate was transferred into 2 liter Double cone blender and lubricated for 5 minutes at 20 rpm.

13. The lubricated blend was compressed with 7 mm, round, standard concave punches using single rotary tablet compression machine.

14. Core tablets were coated using Opadry Brown 03F565239 (Example 3) and Opadry AMB II 88A180040 White (Example 4).

**Table 9. Content uniformity of coated tablets**

|  | Example 3 | Example 4 |
|---|---|---|
| Tablet | Content Uniformity | |
| 1 | 99 | 100.3 |
| 2 | 101.2 | 100.6 |
| 3 | 93.6 | 95.5 |
| 4 | 95.6 | 100.1 |
| 5 | 99.5 | 98.9 |
| 6 | 99.5 | 102.6 |
| 7 | 93.9 | 98.3 |
| 8 | 92.3 | 99.1 |
| 9 | 101.5 | 106.6 |
| 10 | 92.8 | 100.3 |
| **Avg.** | **96.2** | **100.2** |
| **Min** | **3.7** | **95.2** |
| **Max** | **3.85** | **106.6** |
| **SD** | **3.7** | **2.93** |
| **%RSD** | **3.85** | **2.92** |
| **AV** | **11.18** | **7.03** |

**Table 10. Related substances data of coated tablets**

|  | Example 3 | Example 4 |
|---|---|---|
| Imp-A | 0.07 | 0.08 |
| Imp-B | ND | ND |
| Imp-C | ND | 0.04 |
| Imp-D | ND | ND |
| Unspecified impurity | 0.11 | 0.03 |
| Total impurities | 0.18 | 0.15 |

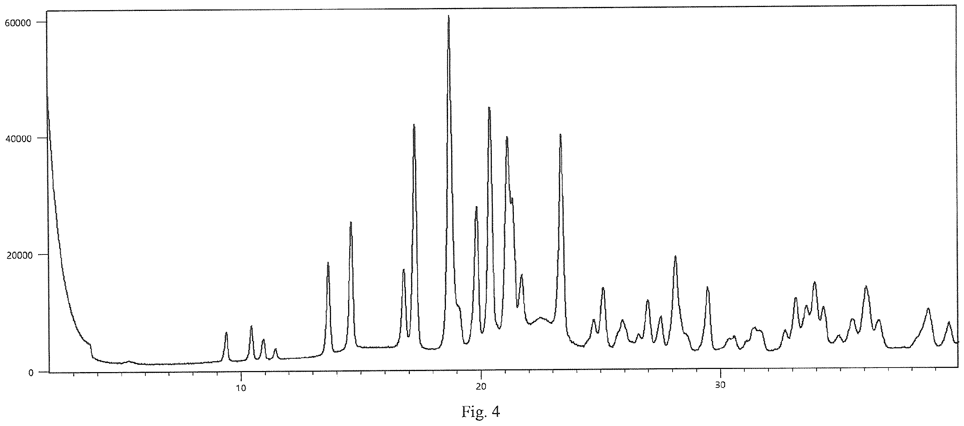

XRD of the coated tablets does not show any peaks for crystalline API (Figure 4: XRD for coated tablet of Example 3). Hence, the API in the formulations is in a non-crystalline state. Content uniformity of the tablets has improved by slugging process. The impurity levels are well controlled.

Examples 5 and 6

| Ingredients | Example 5 [mg] | | Example 6 [mg] | |
|---|---|---|---|---|
| **Stage-A (Hot melt extrusion)** | | | | |
| Selexipag | 1.620 | | 1.620 | |
| Copovidone (Kollidon® VA64) | 4.860 | | 4.860 | |
| Mannitol (Pearlitol® 200SD) | 1.620 | | 1.620 | |
| **Stage-B (Blending)** | | | | |
| Mannitol (Pearlitol® 200SD) | 67.700 | | 67.700 | |
| Cellulose, Microcrystalline (Comprecel® M 102) | 20.000 | | 20.000 | |
| Croscarmellose sodium (Ac-Di-Sol®) | 2.500 | | 2.500 | |
| Colloidal hydrated Silica (Syloid AL1-FP®) | 3.000 | | - | |
| Magnesium aluminometasilicate (Neusilin US2) | - | | 3.000 | |
| **Stage-C (Lubrication for slugging)** | | | | |
| Magnesium stearate (Ligamed MF-2-V) | 0.700 | | 0.700 | |
| **Stage-C (Lubrication for compression)** | | | | |
| Colloidal hydrated Silica (Syloid AL1-FP®) | 2.000 | | - | |
| Magnesium aluminometasilicate (Neusilin US2) | - | | 2.000 | |
| Magnesium stearate (Ligamed MF-2-V) | 1.000 | | 1.000 | |
| **Core tablet weight** | **135.000** | | **135.000** | |
| **Stage-D (Coating)** | | | | |
| | **Ex. 5A** | **Ex. 5B** | **Ex. 6A** | **Ex. 6B** |
| Opadry Brown 03F565239 | 5.000 | - | 5.000 | - |
| Opadry AMB II 88A180040 White | - | 5.000 | - | 5.000 |
| Water, Purified | 36.666 | 20.000 | 36.666 | 20.000 |
| **Coated tablet weight** | **140.000** | **140.000** | **140.000** | **140.000** |

**Procedure:**

1. Accurately dispensed quantity of Selexipag, Copovidone and Mannitol were sifted through mesh #25.
2. The materials of step 1 were blended for 10 minutes.
3. The mixture of step 3 was hot melt extruded by using following screw design and parameters (Fig. 5)

| | Zone temperatures (°C) | | | | | | | | Die Melt (°C) | Die bar | Torque (%) | Screw rpm |
|---|---|---|---|---|---|---|---|---|---|---|---|---|
| | 2 | 3 | 4 | 5 | 6 | 7 | 8 | Die | | | | |
| Set | 40 | 80 | 120 | 135 | 135 | 135 | 135 | 138 | | | | |
| Time (min) | Actual | Actual | Actual | Actual | Actual | Actual | Actual | Actual | | | | |
| Initial | 40 | 80 | 120 | 135 | 135 | 135 | 135 | 138 | 134 | 6 | 20 | 200 |
| 5 | 40 | 80 | 120 | 135 | 135 | 135 | 135 | 138 | 135 | 5 | 24 | 200 |
| 10 | 40 | 80 | 120 | 135 | 135 | 135 | 135 | 138 | 135 | 3 | 19 | 200 |

4. Extrudes were milled using Quadro Comil® through 813 and 457 μ screens at 4000 rpm.

5. Extragranular Mannitol, Cellulose, Microcrystalline, Croscarmellose sodium and Neusilin US2 (Example 6), Syloid AL1-FP (Example 5) were sifted through mesh #40.

6. Magnesium stearate was sifted through mesh #40.

7. Milled extrudes of step 4 along with the materials of step 5 were blended for 10 minutes using Double cone blender.

8. Sifted Magnesium stearate of step 6 was added to the materials of step 7 and lubricated for 5 minutes.

9. Lubricated blend of step 8 was slugged using 16 mm, round, flat punches at 50-70 N hardness.

10. Slugs were milled using Multi mill through 2 mm screen with knives forward direction at slow speed.

11. Milled granules of step10 and sifted Magnesium stearate was transferred into 2 liter Double cone blender and lubricated for 5 minutes at 20 rpm.

12. The lubricated blend was compressed with 7 mm, round, standard concave punches.

13. Core tablets were coated using Opadry Brown 03F565239 (Examples 5A and 6A) and Opadry AMB II 88A180040 White (Examples 5B and 6B).

**Table 11. Content uniformity of core tablets**

| Tablet | Example 5 |
|---|---|
| 1 | 102.9 |
| 2 | 103.2 |
| 3 | 101.1 |
| 4 | 103.7 |
| 5 | 104 |
| 6 | 105.5 |
| 7 | 92.4 |
| 8 | 103.9 |
| 9 | 111.4 |
| 10 | 95.6 |
| **Avg.** | **102.4** |
| **Min** | **92.4** |
| **Max** | **111.4** |
| **SD** | **5.23** |
| **%RSD** | **5.11** |
| **AV** | **13.45** |

**Table 12. Related substances data of core tablets**

| Test | Ex. 5A | Ex. 5B | Ex. 6A | Ex. 6B |
|---|---|---|---|---|
| Imp-A | 0.09 | 0.09 | 0.09 | 0.09 |
| Imp-B | ND | ND | ND | ND |
| Imp-C | ND | ND | ND | ND |
| Imp-D | ND | ND | ND | ND |
| Unspecified impurity | 0.04 | 0.04 | 0.04 | 0.04 |
| Total impurities | 0.15 | 0.15 | 0.15 | 0.15 |

**Table 13. Related substances data of coated tablets after storage at 60°C for 2 days in glass vial**

| Test | Ex. 5A | Ex. 5B | Ex. 6A | Ex. 6B |
|---|---|---|---|---|
| Imp-A | 0.26 | 0.45 | 0.22 | 0.29 |
| Imp-B | ND | ND | ND | ND |
| Imp-C | 0.05 | 0.06 | 0.04 | 0.09 |
| Imp-D | ND | ND | ND | ND |
| Unspecified impurity | 0.04 | 0.09 | 0.04 | 0.04 |
| Total impurities | 0.40 | 0.63 | 0.28 | 0.43 |

**Claims**

1. Process for the preparation of a solid unit dosage form for oral administration containing selexipag in a non-crystalline state, comprising the steps:

   i) subjecting a first mixture containing selexipag, a polymer which is copovidone and optionally a first pharmaceutical excipient to hot-melt extrusion,
   ii) milling the extrudate obtained in step (i) to obtain a milled extrudate,
   iii) mixing the milled extrudate obtained in step (ii) and a second pharmaceutical excipient to obtain a second mixture,
   iv) subjecting the second mixture obtained in step (iii) to compaction,
   v) milling the compacted material obtained in step (iv) to obtain granules,
   vi) optionally mixing the granules obtained in step (v) and a third pharmaceutical excipient to obtain a third mixture, and
   vii) converting the granules obtained in step (v) or the third mixture obtained in step (vi) to the solid unit dosage form.

2. The process according to claim 1, wherein the first pharmaceutical excipient is selected from a sugar alcohol, a monomeric plasticizer and an antioxidant.

3. The process according to claim 1 or 2, wherein the second pharmaceutical excipient is selected from a filler, a disintegrant, a mesoporous inorganic hygroscopic-stability increasing substance and a lubricant.

4. The process according to any one of the preceding claims, wherein the third pharmaceutical excipient is selected from a disintegrant, a mesoporous inorganic hygroscopic-stability increasing substance and a lubricant.

5. The process according to claim 4, wherein:

   - the first mixture of step (i) consists of selexipag, copovidone and optionally a first pharmaceutical excipient which is a sugar alcohol,
   - the second mixture of step (iv) consists of the milled extrudate and the second pharmaceutical excipient which is a filler, a disintegrant, a mesoporous inorganic hygroscopic-stability increasing substance and a lubricant,
- the third mixture of step (vii) consists of the granules and the third pharmaceutical excipient which is a lubricant and optionally a mesoporous inorganic hygroscopic-stability increasing substance.

6. The process according to claim 5, wherein the disintegrant is croscarmellose sodium, the lubricant is magnesium stearate and the mesoporous inorganic hygroscopic-stability increasing substance is selected from silica and magnesium aluminometasilicate.

7. The process according to any one of the preceding claims, wherein the solid unit dosage form for oral administration is an optionally film-coated tablet or a capsule.

8. Solid unit dosage form for oral administration containing selexipag in a non-crystalline state prepared by the process according to any one of the preceding claims.

9. The solid unit dosage form according to claim 8, which is an optionally film-coated tablet prepared by subjecting the granules obtained in step (v) or the third mixture obtained in step (vi) to compression and optionally film-coating, or which is a capsule prepared by filling the granules obtained in step (v) or the third mixture obtained in step (vi) into a capsule.

10. The solid unit dosage form according to claim 9, wherein the granules obtained in step (v) or the third mixture obtained in step (vi) contain the drug in an amount of 2.0 % by weight or less, preferably 0.10-1.5 % by weight, more preferred 0.14-1.2 % by weight.

11. The solid unit dosage form according to claim 9 or 10, wherein the optionally film-coated tablet or the capsule contain the drug in an amount of 200-1600 $\mu$g, preferably 200, 400, 600, 800, 1000, 1200, 1400 or 1600 $\mu$g.

12. Solid unit dosage form for oral administration according to any one of claims 8-11 for use in a method of treating pulmonary arterial hypertension.

**Patentansprüche**

1. Verfahren zur Herstellung einer festen Darreichungsform zu oralen Verabreichung, die Selexipag in einer nicht kristallinen Form enthält, umfassend die Schritte:

i) Durchführung einer Schmelzextrusion mit einem ersten Gemisch, enthaltend Selexipag, ein Polymer, das Copovidon ist, und gegebenenfalls einen ersten pharmazeutischen Hilfsstoff,
ii) Mahlen des in Schritt (i) erhaltenen Extudats, um ein gemahlenes Extrudat zu erhalten,
iii) Mischen des in Schritt (ii) erhaltenen gemahlenen Extrudats und eines zweiten pharmazeutischen Hilfsstoffs, um ein zweites Gemisch zu erhalten,
iv) Kompaktieren des in Schritt (iii) erhaltenen zweiten Gemischs,
v) Mahlen des in Schritt (iv) erhaltenen kompaktierten Materials, um ein Granulat zu erhalten,
vi) gegebenenfalls Mischen des in Schritt (v) erhaltenen Granulats und eines dritten pharmazeutischen Hilfsstoffs, um ein drittes Gemisch zu erhalten, und
vii) Umwandlung des in Schritt (v) erhaltenen Granulats oder des in Schritt (vi) erhaltenen dritten Gemischs in die feste Darreichungsform.

2. Verfahren gemäß Anspruch 1, wobei der erste pharmazeutische Hilfsstoff aus einem Zuckeralkohol, monomeren Weichmacher und einem Antioxidant ausgewählt ist.

3. Verfahren gemäß Anspruch 1 oder 2, wobei der zweite pharmazeutische Hilfsstoff aus einem Verdünnungsmittel, einem Sprengmittel, einer mesoporösen anorganischen, die Hygroskopiestabilität erhöhenden Substanz und einem Schmiermittel ausgewählt ist.

4. Verfahren gemäß einem der vorherstehenden Ansprüche, wobei der dritte pharmazeutische Hilfsstoff aus einem Sprengmittel, einer mesoporösen anorganischen, die Hygroskopiestabilität erhöhenden Substanz und einem Schmiermittel ausgewählt ist.

**5.** Verfahren gemäß Anspruch 4, wobei:

- das erste Gemisch im Schritt (i) aus Selexipag, Copovidon und gegebenenfalls einem ersten pharmazeutischen Hilfsstoff, der ein Zuckeralkohol ist, besteht,
- das zweite Gemisch im Schritt (iv) aus dem gemahlenen Extrudat und dem zweiten pharmazeutischen Hilfsstoff, der ein Verdünnungsmittel, ein Sprengmittel, eine mesoporöse anorganische, die Hygroskopiestabilität erhöhende Substanz und ein Schmiermittel ist, besteht,
- das dritte Gemisch im Schritt (vii) aus dem Granulat und dem dritten pharmazeutischen Hilfsstoff, der ein Schmiermittel und gegebenenfalls eine mesoporöse anorganische, die Hygroskopiestabilität erhöhende Substanz ist, besteht.

**6.** Verfahren gemäß Anspruch 5, wobei das Sprengmittel Croscarmellose-Natrium, das Schmiermittel Magnesiumstearat und die mesoporöse anorganische, die Hygroskopiestabilität erhöhende Substanz aus Siliciumdioxid und Magnesium-Aluminometasilicat ausgewählt ist.

**7.** Verfahren gemäß einem der vorstehenden Ansprüche, wobei die feste Darreichungsform zur oralen Verabreichung eine gegebenenfalls filmbeschichtete Tablette oder eine Kapsel ist.

**8.** Feste Darreichungsform zur oralen Verabreichung, die Selexipag in einer nicht kristallinen Form enthält und nach einem Verfahren gemäß einem der vorstehenden Ansprüche hergestellt ist.

**9.** Feste Darreichungsform gemäß Anspruch 8, die eine gegebenenfalls filmbeschichtete Tablette ist und hergestellt ist, indem das in Schritt (v) erhaltene Granulat oder das in Schritt (vi) erhaltene dritte Gemisch komprimiert und gegebenenfalls filmbeschichtet wird, oder die eine Kapsel ist und hergestellt ist, indem das in Schritt (v) erhaltene Granulat oder das in Schritt (vi) erhaltene dritte Gemisch in eine Kapsel gefüllt wird.

**10.** Feste Darreichungsform gemäß Anspruch 9, wobei das in Schritt (v) erhaltene Granulat oder das in Schritt (vi) erhaltene dritte Gemisch den Wirkstoff in einer Menge von 2,0 Gew.-% oder weniger, vorzugsweise 0,10-1,5 Gew.-%, mehr bevorzugt 0,14-1,2 Gew.-%, enthält.

**11.** Feste Darreichungsform gemäß Anspruch 9 oder 10, wobei die gegebenenfalls filmbeschichtete Tablette oder Kapsel den Wirkstoff in einer Menge von 200-1600 $\mu$g, vorzugsweise 200, 400, 600, 800, 1000, 1200, 1400 oder 1600 $\mu$g enthält.

**12.** Feste Darreichungsform zur oralen Verabreichung gemäß einem der Ansprüche 8-11 zur Verwendung in einem Verfahren zur Behandlung der pulmonalarteriellen Hypertonie.

**Revendications**

**1.** Procédé de préparation d'une forme posologique unitaire solide pour une administration orale contenant du sélexipag à l'état non cristallin, comprenant les étapes consistant à :

i) soumettre un premier mélange contenant du sélexipag, un polymère qui est la copovidone et facultativement un premier excipient pharmaceutique à une extrusion à l'état fondu à chaud,
ii) broyer l'extrudat obtenu à l'étape (i) pour obtenir un extrudat broyé,
iii) mélanger l'extrudat broyé obtenu à l'étape (ii) et un deuxième excipient pharmaceutique pour obtenir un deuxième mélange,
iv) soumettre le deuxième mélange obtenu à l'étape (iii) à un compactage,
v) broyer le matériau compacté obtenu à l'étape (iv) pour obtenir des granulés,
vi) facultativement mélanger les granulés obtenus à l'étape (v) et un troisième excipient pharmaceutique pour obtenir un troisième mélange, et
vii) convertir les granulés obtenus à l'étape (v) ou le troisième mélange obtenu à l'étape (vi) en la forme posologique unitaire solide.

**2.** Procédé selon la revendication 1, dans lequel le premier excipient pharmaceutique est choisi parmi un alcool de sucre, un plastifiant monomère et un antioxydant.

**3.** Procédé selon la revendication 1 ou 2, dans lequel le deuxième excipient pharmaceutique est choisi parmi une charge, un désintégrant, une substance minérale mésoporeuse augmentant la stabilité hygroscopique et un lubrifiant.

**4.** Procédé selon l'une quelconque des revendications précédentes, dans lequel le troisième excipient pharmaceutique est choisi parmi un désintégrant, une substance minérale mésoporeuse augmentant la stabilité hygroscopique et un lubrifiant.

**5.** Procédé selon la revendication 4, dans lequel :

- le premier mélange de l'étape (i) est constitué de sélexipag, de copovidone et facultativement d'un premier excipient pharmaceutique qui est un alcool de sucre,
- le deuxième mélange de l'étape (iv) est constitué de l'extrudat broyé et du deuxième excipient pharmaceutique qui est une charge, un désintégrant, une substance minérale mésoporeuse augmentant la stabilité hygroscopique et un lubrifiant,
- le troisième mélange de l'étape (vii) est constitué des granulés et du troisième excipient pharmaceutique qui est un lubrifiant et facultativement une substance minérale mésoporeuse augmentant la stabilité hygroscopique.

**6.** Procédé selon la revendication 5, dans lequel le désintégrant est la croscarmellose sodique, le lubrifiant est le stéarate de magnésium et la substance minérale mésoporeuse augmentant la stabilité hygroscopique est choisie parmi la silice et l'aluminométasilicate de magnésium.

**7.** Procédé selon l'une quelconque des revendications précédentes, dans lequel la forme posologique unitaire solide pour une administration orale est un comprimé facultativement pelliculé ou une capsule.

**8.** Forme posologique unitaire solide pour une administration orale contenant du sélexipag à l'état non cristallin préparée par le procédé selon l'une quelconque des revendications précédentes.

**9.** Forme posologique unitaire solide selon la revendication 8, qui est un comprimé facultativement pelliculé préparé en soumettant les granulés obtenus à l'étape (v) ou le troisième mélange obtenu à l'étape (vi) à une compression et facultativement un pelliculage, ou qui est une capsule préparée en remplissant une capsule avec les granulés obtenus à l'étape (v) ou le troisième mélange obtenu à l'étape (vi).

**10.** Forme posologique unitaire solide selon la revendication 9, dans laquelle les granulés obtenus à l'étape (v) ou le troisième mélange obtenu à l'étape (vi) contiennent le médicament en une quantité de 2,0 % en poids ou moins, de préférence de 0,10 à 1,5 % en poids, de manière plus préférée de 0,14 à 1,2 % en poids.

**11.** Forme posologique unitaire solide selon la revendication 9 ou 10, dans laquelle le comprimé facultativement pelliculé ou la capsule contient le médicament en une quantité de 200 à 1 600 $\mu$g, de préférence de 200, 400, 600, 800, 1 000, 1 200, 1 400 ou 1 600 $\mu$g.

**12.** Forme posologique unitaire solide pour une administration orale selon l'une quelconque des revendications 8 à 11 à utiliser dans un procédé de traitement de l'hypertension artérielle pulmonaire.

Fig. 1

Fig. 2

Fig. 3

Fig. 4

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2447254 A
- WO 2017029594 A
- WO 2017121806 A
- WO 2018078383 A
- WO 2018015974 A

**Non-patent literature cited in the description**

- *Australian Public Assessment Report for Selexipag,* November 2016
- database. 618027